# EUROPEAN PATENT SPECIFICATION

(11) **EP 2 925 286 B1**
(45) Date of publication and mention of the grant of the patent: **31.08.2016**
(21) Application number: 13789556.1
(22) Date of filing: 12.11.2013
(51) Int. Cl.: A61K 8/49, A61Q 5/00, A61K 8/81, A61K 8/23

(54) **HAIR CARE COMPOSITION**
HAARPFLEGEZUSAMMENSETZUNG
COMPOSITION DE SOIN CAPILLAIRE

(30) Priority: 29.11.2012 WO PCT/CN2012/085551; 14.01.2013 EP 13151136
(43) Date of publication of application: 07.10.2015
(73) Proprietor: Unilever PLC, London, Greater London EC4Y 0DY (GB); Unilever N.V., 3013 AL Rotterdam (NL)
(72) Inventor: DAWSON, Joanna, Susan, Wirral Merseyside CH63 3JW (GB); HENTRICH, Doreen, 14471 Potsdam (DE); JARVIS, Adam, Peter, Wirral Merseyside CH63 3JW (GB); KITA-TOKARCZYK, Katarzyna, Elzbieta, 65812 Bad Soden am Taunus (DE); LIMER, Adam, John, Shanghai 200335 (CN)
(74) Representative: James, Helen Sarah
(86) International application number: PCT/EP2013/073571
(87) International publication number: WO 2014/082846

(56) References cited:
- WO-A1-03/088957
- WO-A1-2004/035015
- WO-A1-2010/039145
- WO-A1-2012/035053
- FR-A1- 2 920 975
- US-A- 4 892 916

## Description

The present invention relates to hair care compositions in which a particulate antidandruff agent is selectively deposited to the scalp.

Particulate antidandruff agents, in particular the antidandruff agent zinc pyrithione (Zn-PTO), are commonly used in hair care compositions.

WO 03/088957 discloses a shampoo composition comprising a zinc salt, sodium lauryl sulphate, sodium laureth sulphate and sodium cocoisethionate.

WO 2004/035015 discloses a shampoo comprising an anti-dandruff zinc salt, sodium laureth sulphate and conjugated linoleic acid.

However deposition of particulate antidandruff agents onto the hair from anti-dandruff shampoos can lead to the perception of poor hair conditioning performance. It is therefore beneficial if these agents are delivered to the scalp rather than to the hair. This means that the problem area is treated without affecting the feel and properties of the hair.

Hydrophically-modified Alkali Swellable Emulsion (HASE) polymers such as Aculyn 28 ex Rohm & Haas or Carbopol Aqua SF1 ex-Lubrizol can be used to thicken products. These polymers comprise a polyacrylate backbone, whose solubility is controlled by pH, and hydrophobic pendant groups that associate with surfactant micelles to form a network. Such networks are typically optically transparent and hence offer a combination of clarity and suspending power. However it has been found that such formulations containing such polymers are unable to suspend particulate antidandruff agents such as zinc pyrithione.

The present invention relates to compositions that selectively deposit particulate antidandruff agents onto the scalp in preference to the hair. Furthermore the present invention has the additional advantage that formulations containing particulate antidandruff agents and HASE polymers can be easily formulated.

The present invention relates to an aqueous hair care composition comprising:
i) at least 0.01 wt. % of the total composition of a particulate antidandruff agent;
ii) at least 0.05 wt. % of the addition polymerization product of:
   a) from 0.1 to 5 wt. % of a first unsaturated monomer A of a ethylenically unsaturated diacid of formula (I):

      HOOC-CR₅=CR₆-COOH (I)

      or an unsaturated cyclic anhydride precursor of such an ethylenically unsaturated cis diacid, the anhydride having formula (II) 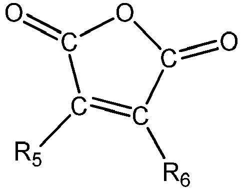 where R₅ and R₆ are individually selected from H, C₁-C₃ alkyl, phenyl, chlorine and bromine;
   b) from 1 to 60 wt. % of a second ethylenically unsaturated monomer B selected from acrylic acid, methacrylic acid and combinations thereof,
   c) from 30 to 75 wt. % of a (meth)acrylate monomer C selected from C₁ to C₈ alkyl esters of (meth)acrylic acid and C₁ to C₈ alkyl esters of methacrylic acid and combinations thereof
   d) from 1 to 25 wt. % of an associative monomer D of the formula III: 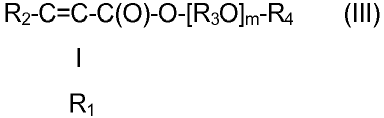
      where R₁ and R₂ are each independently selected from H, and C₁₋₃
      alkyl; R₃ is C₂-C₄ and mixtures thereof, preferably C₂;
      m, the average number of alkoxy units R₃O, is from 6 to 40;
      R₄ is alkyl or alkylaryl where the alkyl part is linear or branched;
      and the total number of carbons is from 6 to 40.

The invention further relates to the use of the above-mentioned copolymer to deposit a particulate antidandruff agent to the scalp and to minimize deposition of the particulate antidandruff agent to the hair.

### Detailed Description of the Invention

### Copolymers

The copolymers of the invention are based on a combination of monomers that include an ethylenically unsaturated polyacidic monomer such as a maleic derivative, a monoacid ethylenically unsaturated monomer such as an acrylic or methacrylic acid, an acrylate ester monomer and an associative monomer.

Preferably the level of copolymer in the invention is from 0.05 to 1 wt. % of the total composition; more preferably from 0.1 to 0.7 wt. %, most preferably from 0.2 to 0.5 wt. %.

Preferably the copolymer is swollen, more preferably fully swollen in an aqueous solution. The term swollen means an increase in the structured volume of a solution associated with the uptake of a liquid. The term fully swollen means that no further liquid can be uptaken (and hence no increase in viscosity occurs). To obtain such a fully swollen polymer it is preferable if the polymer is dissolved in water and the pH increased until no further change in viscosity occurs. The fully swollen polymer can then be added to the formulation or the formulation is built around the fully swollen polymer. It is preferred if the polymer is swollen before addition of the surfactant. It is preferable if ranges of the monomeric units in the polymer are as follows:
Monomer A ranges from 0.2 to 1 % by weight of the copolymer;
Monomer B ranges from 25 to 50 % by weight of the copolymer;
Monomer C ranges from 30 to 65 % by weight of the copolymer; and
Monomer D ranges from 2 to 12 % by weight of the copolymer.
In the context of the present invention the weight % of monomer within the copolymer relates to the non-neutralized monomer (not in salt form).

Preferably the weight ratio of monomer A to monomer D is from 1:3 to 1:30; more preferably from 1:5 to 1:25; most preferably from 1:7 to 1:22. These weight ratios are particularly advantageous with the preferred monomers of A and D listed below.

Preferably the copolymer include neutralized (de-protonated) and partially neutralized forms.

### Monomer A

monomer A is a ethylenically unsaturated cdiacid, preferably a cis diacid of formula (I):

HOOC-CR₅=CR₆-COOH (I)

or an unsaturated cyclic anhydride precursor of such an ethylenically unsaturated cis diacid, the anhydride having formula (II) 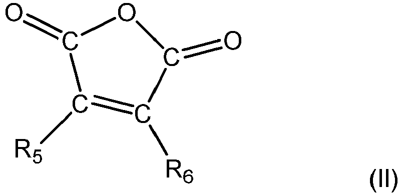 where R₅ and R₆ are individually selected from H, C₁-C₃ alkyl, phenyl, chlorine and bromine;

Preferably the copolymer will include a first ethylenically unsaturated monomer which may be maleic, fumaric, itaconic and citraconic acids and anhydrides thereof as well as combinations of these. Most preferred are maleic derivatives. Accordingly, the most preferred species are maleic acid, maleic anhydride or a combination thereof. Preferably maleic acid can be generated from maleic anhydride as starting material and hydrolyzing this to the di-acid in the emulsion polymerization.

While both fumaric and itaconic acid are contemplated for use herein, in one or more embodiments, it is preferred the itaconic acid and, in further embodiments, both itaconic and fumaric acid, are absent from the subject copolymers or are individually present in only minor amounts, i.e., less than 0.05 wt. %, preferably less than 0.005 wt. %, based on the total weight of monomer. While both fumaric and itaconic acid are contemplated for use herein, in one or more embodiments, it is preferred the itaconic acid and, in further embodiments, both itaconic and fumaric acid, are absent from the subject copolymers or are individually present in only minor amounts, i.e., less than 0.05 wt. %, preferably less than 0.005 wt. %, based on the total weight of monomer.

The first ethylenically unsaturated acid monomer may be employed in amounts of from 0.1 to 5 wt. %, preferably from 0.2 to 4 wt. %, more preferably from 0.2 to 1 wt. %, and, in one or more embodiments, from 0.3 to 0.6 wt. %, based on the total weight of monomer. In one or more embodiments of particular interest, maleic acid accounts for at least 50 % by weight, more preferably at least 80 % by weight, even more preferably, at least 95 % by weight, based on the total weight of the first ethylenically unsaturated acid monomer.

### Monomer B

The second ethylenically unsaturated acid monomer B is selected from acrylic acid, methacrylic acid and combinations thereof.

The second ethylenically unsaturated acid monomer may be employed in amounts of from 15 to 60 wt. %, preferably from 20 to 55 wt. %, more preferably from 25 to 50 wt. %, based on total monomer. In one or more embodiments of interest the amount of second ethylenically unsaturated acid monomer is from 40 to 50 wt. % based on total monomer; in one or more other embodiments of interest, the amount of second ethylenically unsaturated acid monomer is from 20 to 40 wt. % based on total monomer. In one or more embodiments of particular interest methacrylic acid accounts for at least 50 % by weight, more preferably at least 70 % by weight, even more preferably, at least 90 % by weight, of the total weight of the second ethylenically unsaturated acid monomer.

### Monomer C

The (meth)acrylate monomer C may be selected from C₁ to C₈ alkyl esters of (meth)acrylic acid and C₁ to C₈ alkyl esters of methacrylic acid and combinations thereof, with C₁ to C₄ alkyl esters of such acids, being particularly preferred. Preferred ester monomers are ethyl acrylate, methyl acrylate, ethyl methacrylate, methyl methacrylate, butyl acrylate, and butyl methacrylate, ethyl acrylates is particularly preferred. In one or more embodiments of particular interest, C₁ to C₄ alkyl esters of acrylic acid, preferably ethyl acrylate, account, for at least 50 % by weight, more preferably at least 70 % by weight, even more preferably, at least 90 % by weight of the (meth)acrylate monomer.

### Monomer D

D is preferably an associative monomer of formula (IV) 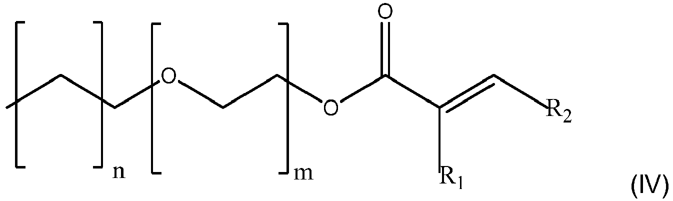 in which each R₁ and R₂ are independently selected from H, C₁ to C₃ alkyl.

Preferably
R₁ is a methyl group and R₂ is H.
n ranges from 6 to 40 and m ranges from 6 to 40, more preferably n ranges from 10 to 30 and m ranges 15 to 35 most preferably n ranges from 12 to 22 and m ranges from 20 to 30. It is preferable if m is greater or equal to n.

The associative monomer may be employed in amounts of from 1 to 25 wt. %, preferably from 2 to 20 wt. %, and more preferably from 2 to 12 wt. %, based on total monomer. In one or more embodiments of particular interest the amount of associate monomer employed is from 5 to 10 wt. %, based on total monomer.

Preferably the polymer is at least 0.05 wt. % of the addition polymerization product of:
a) from 0.1 to 5 wt. % of a first ethylenically unsaturated monomer selected from maleic, fumaric, itaconic and citraconic acid, their anhydride precursors and combinations thereof; and
b) from 1 to 60 wt. % of a second ethylenically unsaturated monomer B selected from acrylic acid, methacrylic acid and combinations thereof,
c) from 30 to 75 wt. % a (meth)acrylate monomer C selected from C₁ to C₈ alkyl esters of (meth)acrylic acid and C₁ to C₈ alkyl esters of methacrylic acid and combinations thereof,
d) from 1 to 25 wt. % of an associative monomer D of the formula:

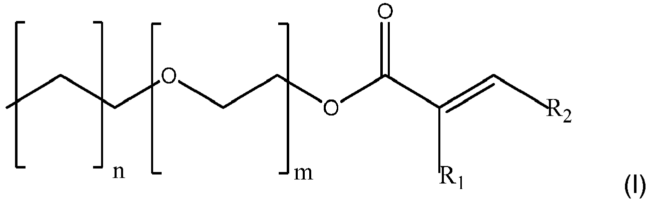

in which each R₁ and R₂ are independently H, C₁ to C₃ alkyl
n ranges from 6 to 40 and m ranges from 6 to 40.

In some embodiments, one or more of the acid groups of the starting acid monomers may be neutralized to salt form. Typical salt counter-ions to the acid groups are alkali metals, especially sodium and potassium, and ammonium and triethanolammonium cations.

### Particulate Antidandruff Anent

Compositions of the invention comprise a particulate antidandruff agent.

Preferred antidandruff agents comprise selenium or zinc. If a selenium salt is used it is preferred if it is selenium sulphide. The anti-dandruff is preferably a zinc salt and is more preferably selected from zinc pyrithione, zinc sulfate and hydrates thereof (e.g. zinc sulfate hexahydrate), and combinations. Zinc pyrithione (ZnPTO) which is an alternate name for zinc 1-hydroxy-2-pyridinethione is preferred.

The average particulate size of the particulate antidandruff agent, in particular metal salt is preferably less than 10 micrometers, more preferably from 0.1 micrometers to 5 micrometers.

The antidandruff agent, preferably zinc pyrtithione is preferably present at a level of from 0.05 to 5 wt. %, preferably from 0.1 to 3 wt. %, more preferably from 0.25 to 2.5 wt. % based on the total weight of the composition.

### Other Ingredients

In a highly preferred form compositions in accordance with the invention are formulated as compositions for washing the hair and subsequent rinsing.

### Cleansing Surfactant

Shampoo compositions of the invention comprise one or more cleansing surfactants which are cosmetically acceptable and suitable for topical application to the hair. Further surfactants may be present as emulsifiers.

Suitable cleansing surfactants are selected from anionic, amphoteric and zwitterionic surfactants, and mixtures thereof. The cleansing surfactant may be the same surfactant as the emulsifier, or may be different.

Compositions of the invention comprise anionic surfactant. Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae R₂OSO₃M and R₁O(C₂H₄O)ₓSO₃M, wherein R₂ is alkyl or alkenyl of from 8 to 18 carbon atoms, x is an integer having a value of from about 1 to about 10, and M is a cation such as ammonium, alkanolamine, such as triethanolamine, monovalent metal, such as sodium and potassium, and polyvalent metal cation, such as magnesium, and calcium. Most preferably R₂ has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably the level of alkyl ether sulphate is from 0.5 wt. % to 25 wt. % of the total composition, more preferably from 3 wt. % to 18 wt. %, most preferably from 6 wt. % to 15 wt. % of the total composition.

The total amount of anionic cleansing surfactant in compositions of the invention generally ranges from 0.5 wt. % to 45 wt. %, more preferably from 1.5 wt. % to 20 wt. %.

Compositions of the invention may comprise fatty acyl isethionate, if present preferably at a level of from 1 to 10 wt. %, more preferably from 2 to 8 wt. %, most preferably from 2.5 to 7.5 wt. %.

A preferred fatty acyl isethionate product comprises fatty acyl isethionate surfactant at a level of from 40 to 80 wt. % of the product, as well as free fatty acid and/or fatty acid salt at a level of from 15 to 50 %.

Preferably, greater than 20 wt. % and less than 45 wt. %, more preferably greater than 25 wt. % and less than 45 wt. % of the fatty acyl isethionate are of chain length greater than or equal to C₁₆; and greater than 50 wt. %, preferably greater than 60 wt. % of the free fatty acid/soap is of chain length C₁₆ to C₂₀.

In addition, the product may contain isethionates salts which are present typically at levels less than 5 wt. %, and traces (less than 2 wt. %) of other impurities.

Preferably, a mixture of aliphatic fatty acids is used for the preparation of commercial fatty acyl isethionates surfactants. The resulting fatty acyl isethionate surfactants (e.g., resulting from reaction of alkali metal isethionate and aliphatic fatty acid) preferably should have more than 20 wt. %, preferably more than 25 wt. %, but no more than 45 wt. %, preferably 35 % (on basis of fatty acyl isethionates reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

Examples of commercial fatty acyl isethionate products that are particularly useful in the subject invention are DEFI flakes and Dove^{®} cleansing bar noodles produced by Unilever. DEFI (Direct Esterification of Fatty Isethionate) flakes typically contain about 68 to 80 wt. % of sodium fatty acyl isethionate and 15 to 30 wt. % free fatty acid. More than 25 wt. % and no more than 35 % of fatty acyl group of the resulting fatty acyl isethionate have 16 to 18 carbon atoms. Dove^{®} cleansing bar noodles are mixtures of DEFI flakes described above and long chain (mainly C₁₆ and C₁₈) fatty acid and fatty soap which contain about 40 to 55 wt. % of fatty acyl isethionate and 30 to 40 wt. % of fatty acid and fatty soap.

Compositions of the invention may contain non-ionic surfactant. Most preferably non-ionic surfactants are present in the range 0 to 5 wt. %.

Nonionic surfactants that can be included in compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH₂CH₂)ₙOH, where R is an alkyl chain of C12 to C15, and n is 5 to 9.

Other suitable nonionic surfactants include mono- or di-alkyl alkanolamides. Examples include coco mono- or di-ethanolamide and coco mono- isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

Amphoteric or zwitterionic surfactant can be included in an amount ranging from 0.5 wt. % to about 8 wt. %, preferably from 1 wt. % to 4 wt. % of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

### Silicone Conditioning Agents

The compositions of the invention contain emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cSt at 25°C the viscosity of the silicone itself is preferably at least 60,000 cSt, most preferably at least 500,000 cSt, ideally at least 1,000,000 cSt. Preferably the viscosity does not exceed 10⁹ cSt for ease of formulation.

Emulsified silicones for use in the shampoo compositions of the invention will typically have an average silicone droplet size in the composition of less than 30, preferably less than 20, more preferably less than 10 micrometers, ideally from 0.01 to 1 micrometers. Silicone emulsions having an average silicone droplet size of less than 0.15 micrometers are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

Combination of amino and non amino functional silicones may also be used.

The total amount of silicone is preferably from 0.01 wt. % to 10 wt. % of the total composition more preferably from 0.1 wt. % to 5 wt. %, most preferably 0.5 wt. % to 3 wt. %.

### Cationic Deposition Polymer

Cationic polymers may be present in the composition of the invention for further enhancing deposition performance.

Suitable cationic polymers may be homopolymers which are cationically substituted or may be formed from two or more types of monomers. The weight average (M_{w}) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual, R is an alkylene, oxyalkylene, polyoxyalkylene, or hydroxyalkylene group, or combination thereof, R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 to 5 %, preferably from 0.05 to 1 %, more preferably from 0.08 to 0.5 % by total weight of cationic polymer based on the total weight of the composition.

### Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof.

Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include
paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols. Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt. %.

### Further Optional Ingredients

The compositions of the present invention may also contain adjuncts suitable for hair care. Generally such ingredients are included individually at a level of up to 2, preferably up to 1 wt. % of the total composition.

Suitable hair care adjuncts include perfumes, fibre actives, amino acids, sugars, preservatives, pH adjusters and ceramides.

Preferably salt is present at levels from 0.1 to 1 wt. % of the total composition to adjust the product viscosity.

Preferably NaOH is present at levels from 0.1 to 1 wt. % of the total composition to adjust the pH of the formulation.

The invention will be illustrated by the following non-limiting Examples.

### Examples

### Example 1

### Associative Monomer Synthesis

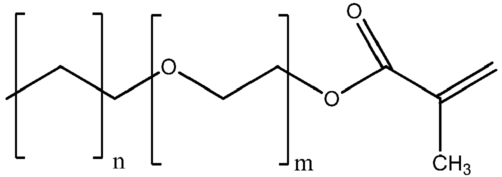

n=12
m = 23

Brij® 35P (150g) was dissolved in 500ml anhydrous dichloromethane under a nitrogen atmosphere and cooled in an ice bath to 5°C. Triethylamine (18.6g) was added via syringe before methacryloyl chloride (20.9g) was added dropwise over a 30 minute period. After complete addition, the solution was allowed to warm to room temperature and the reaction stirred for 4 weeks. The solution was then filtered to remove the resulting precipitate and washed once with saturated sodium hydrogen carbonate solution (200ml) and once with saturated brine (200ml). The solution was then passed through a column containing basic alumina before the product was dried with anhydrous magnesium sulphate, filtered and the solvent removed *in vacuo.* In subsequent examples the product is referred to as Surfmer D1.

### HASE copolymer synthesis

A round bottom flask was charged with ethyl acrylate (66.8g), methacrylic acid (37.7g), maleic anhydride (0.515g) and SurmerD1 (10.0g). The mixture was sealed and purged with nitrogen for 60 minutes before sodium dodecyl sulfonate (1.03 g) and deoxygenated water (26.5g) was added and stirred forming a pre-emulsion. A multineck round bottom flask was fitted with a nitrogen sparge and overhead stirrer. Deoxygenated water (181g) and sodium dodecyl sulfonate (0.298g) were added, stirred at 250 rpm and heated to 90°C. Ammonium persulfate (0.073g) in water (1 ml) was added via syringe. The pre-emulsion was fed into the surfactant solution via peristaltic pump over 150 minutes. After complete addition, ammonium persulfate (0.033g) in water (1 ml) was added and the reaction stirred for a further 240 minutes. Copolymers in Table x were synthesised by using suitable adaptations of this process.

**Table 1**

| | **Raw Material Charge (Weight in Grams)** | | | |
|---|---|---|---|---|
| **Component** | Polymer 1.1 | Polymer 1.2 | Polymer 1.3 | Polymer 1.4 |
| **Pre-emulsion** | | | | |
| Ethyl Acrylate | 66.8 | 74.0 | 70.7 | 54.2 |
| Methacrylic Acid | 37.7 | 41.7 | 43.1 | 30.6 |
| Maleic anhydride | 0.515 | 0.57 | 0.71 | 0.506 |
| Surfmer D1 | 10.0 | 4.26 | 7.91 | 8.12 |
| Water | 26.5 | 29.3 | 36.0 | 21.5 |
| Sodium Dodecyl Sulphonate | 1.03 | 1.14 | 1.18 | 0.84 |

| **Surfactant solution** | | | | |
|---|---|---|---|---|
| Deoxygenated Water | 181.0 | 200.6 | 207.3 | 147.1 |
| Sodium Dodecyl Sulphonate | 0.298 | 0.33 | 0.341 | 0.242 |
| Ammonium Persulfate Solution | 0.073 | 0.081 | 0.084 | 0.059 |

| **Post Addition** | | | | |
|---|---|---|---|---|
| Ammonium Persulfate Solution | 0.033 | 0.036 | 0.037 | 0.026 |

**Table 2**

| Polymer composition | MAA w% | MA w% | EA w% | Surfmer w% |
|---|---|---|---|---|
| 1.1 | 32.8 | 0.45 | 58.1 | 8.7 |
| 1.2 | 34.6 | 0.47 | 61.4 | 3.5 |
| 1.3 | 35.2 | 0.58 | 57.7 | 6.5 |
| 1.4 | 32.7 | 0.54 | 58.0 | 8.7 |

The resulting copolymers were used in the Examples below.

### Examples

Formulations containing anti-dandruff active Zn-PTO were prepared with the copolymer Example 1 at different levels, Examples 1.1, and 1.2 compared to a similar shampoo formulation comprising Carbopol 980 as shown in Example A.

### Preparation of Examples

The polymer was added to water, and then NaOH is added. The polymer swells in water and produces intramolecular hydrophobic associations. The surfactant was added after swelling. Jaguar was then added to the formulation followed by the remaining ingredients. Finally the pH was adjusted and salt was added to adjust the viscosity.

**Table 3**

| | | **Example %W/W** | | | | | | | |
|---|---|---|---|---|---|---|---|---|---|
| **Chemical name** | **Tradename** | **A** | **B** | **C** | **1.1** | **1.2** | **1.3** | **1.4** | **1.5** |
| Sodium Laureth Sulfate | Texapon N701 | 12.0 | 12.0 | 12.0 | 12.0 | 12.0 | 12.0 | 12.0 | 12. 0 |
| Cocamidopropyl Betaine | Tegobetaine CK | 1.6 | 1.6 | 1.6 | 1.6 | 1.6 | 1.6 | 1.6 | 1.6 |
| Structuring Polymer | Carbopol 980 | 0.6 | - | - | - | - | - | - | - |
| Structuring Polymer | Aculyn 28 | - | 0.6 | - | - | - | - | - | - |
| Structuring Polymer | Carbopol Aqua SF1 | - | - | 0.5 | - | - | - | - | - |
| Structuring Polymer | Example 1.1 Copolymer | - | - | - | 0.2 | 0.4 | - | - | - |
| Structuring Polymer | Example 1.2 Copolymer | - | - | - | - | - | 0.4 | - | - |
| Structuring Polymer | Example 1.3 Copolymer | - | - | - | - | - | - | 0.4 | - |
| Structuring Polymer | Example 1.4 Copolymer | - | - | - | - | - | - | - | 0.4 |
| Zinc Pyrithione | Zinc Omadine FPS | 2.1 | 2.1 | 2.1 | 2.1 | 2.1 | 2.1 | 2.1 | 2.1 |
| Zinc Sulfate Heptahydrate | Zinc Sulfate Heptahydrat e | 0.1 | 0.1 | 0.1 | 0.1 | 0.1 | 0.1 | 0.1 | 0.1 |
| Dimethiconol/C12-15 Pareth | Silicone Emulsion DC5-7128 | 1.5 | 1.5 | 1.5 | 1.5 | 1.5 | 1.5 | 1.5 | 1.5 |
| Dimethiconol / TEA-dodecylbenzene sulfonate | Silicone emulsion DC-1788 | 2.6 | 2.6 | 2.6 | 2.6 | 2.6 | 2.6 | 2.6 | 2.6 |
| Guar Hydroxypropyltrimonium Chloride | BFG-Jaguar C17 | 0.2 | 0.2 | 0.2 | 0.2 | 0.2 | 0.2 | 0.2 | 0.2 |
| NaCl | | As require for pH and viscosity | | | | | | | |
| NaOH | | | | | | | | | |
| Water and minors | Water and minors | q.s.to 100 | | | | | | | |
| pH | | 5.5-6.5 | | | | | | | |

All products had viscosities adjusted between 2,000 cP and 10,000 cP at 25°C using a Brookfield viscometer at 20 rpm(spindle 5).

Examples B and C immediately phase separated and it was not possible to measure the zinc deposition.

### Zinc Deposition Analysis (on bioskin)

For each product, five repetitions were competed. They were treated using the following method:
1. 1.5 ml of water was added into the plastic ring.
2. 0.5 ml of shampoo was then added into the ring.
3. The shampoo was applied onto the entire surface of artificial skin.
4. The shampoo solution was removed.
5. The artificial skin was rinsed with rubbing with 2 ml of water for 30 seconds.
6. The rinsing water was removed.
7. Samples were left to dry overnight then subjected XRF analysis

Zinc deposition on hair:
2.5 g 6 inch switches of Dark Brown European hair (straight) was used.

### (1) Base wash

∘ 2x base washed with 14 % SLES using 0.1 g/g Sles/Hair = 1.25 g of SLES on 5 switches simultaneously
o Wet hair in 37°C 4 L/min water from constant flow tap (10 sec)
o SLES applied
o Massaged 30 sec
o Rinsed 30 sec
o SLES wash repeated
o Combed to remove tangles

### (2) Shampoo wash

o Shampoo applied (1.25 g on 5 switches)
o Massaged 30 sec
o Rinsed 30 sec
o Shampoo wash repeated

### (3) Drying + cells preparation

o Left to dry hanging in drying cabinet at 50°C
o Mounted onto XRF cells and trim excess hair

### (4) XRF analysis

**Table 4**

| **Structurant** | **Zinc deposition on hair (average, ppm)** | **Zinc deposition on bioskin (average, ppm)** |
|---|---|---|
| Example A (with 0.6% Carbopol 980) | 1194 | 250 |
| Example 1.1 (with 0.2% of Example 1 Copolymer) | 400 | 250 |
| Example 1.2 (with 0.4% of Example 1.1 Copolymer) | 573 | 230 |
| Example 1.3 (with 0.4 % of copolymer 1.2) | 437 | 300 |
| Example 1.4 (with 0.4 % of copolymer 1.3) | 569 | 230 |
| Example 1.5 (with 0.4 % of copolymer 1.4) | 610 | 300 |

| | | |
|---|---|---|
| The Examples of the invention demonstrate that Zn-PTO deposits on bioskin yet Zn-PTO deposition on hair is inhibited. | | |

## Claims

1. An aqueous hair care composition comprising:
i) at least 0.01 wt. % of the total composition of a particulate antidandruff agent;
ii) at least 0.05 wt. % of the addition polymerization product of:
a) from 0.1 to 5 wt. % of a first unsaturated monomer A of a ethylenically unsaturated diacid of formula (I):
HOOC-CR₅=CR₆-COOH (I)
or an unsaturated cyclic anhydride precursor of such an ethylenically unsaturated cis diacid, the anhydride having formula (II) 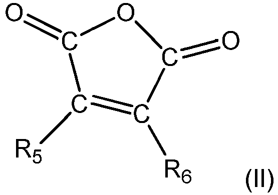 where R₅ and R₆ are individually selected from H, C₁-C₃ alkyl, phenyl, chlorine and bromine;
b) from 1 to 60 wt. % of a second ethylenically unsaturated monomer **B** selected from acrylic acid, methacrylic acid and combinations thereof,
c) from 30 to 75 wt. % of a (meth)acrylate monomer **C** selected from C₁ to C₈ alkyl esters of (meth)acrylic acid and C₁ to C₈ alkyl esters of methacrylic acid and combinations thereof
d) from 1 to 25 wt. % of an associative monomer **D** of the formula III: 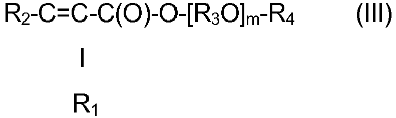
Where R₁ and R₂ are each independently selected from H, and 6₁₋₃ alkyl; R₃ is C₂-C₄ and mixtures thereof, preferably C₂;
m, the average number of alkoxy units R₃O, is from 6 to 40;
R₄ is alkyl or alkylaryl where the alkyl part is linear or branched; and the total number of carbons is from 6 to 40.

2. A composition according to any preceding claim in which the monomer A is selected from the group consisting of maleic anhydride, maleic acid and combinations thereof.

3. A composition according to any preceding claim in which the monomer B is methacrylic acid.

4. A composition according to any preceding claim in which monomer C of the copolymer is selected from the group consisting of ethylacrylate, methylacrylate, ethylmethacrylate, methylmethacrylate, butylacrylate, butylmethacrylate and mixtures thereof.

5. A composition according to any preceding claim in which monomer C of the copolymer is ethylacrylate.

6. A composition according to any preceding claim in which monomer D is of formula iv 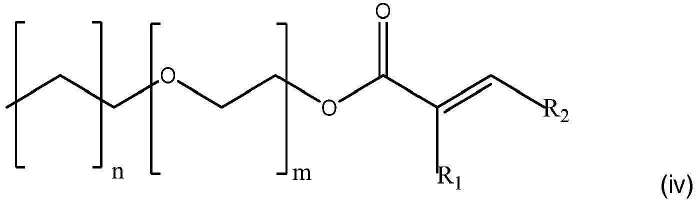 in which each R₁ and R₂ are independently H, C₁ to C₃ alkyl; n ranges from 6 to 40 and m ranges from 6 to 40.

7. A composition according to any preceding claim in which for monomer D n ranges from 12 to 22 and m ranges from 20 to 30.

8. A composition according to any preceding claim in which in monomer D is R₁ is H or methyl and R₂ is H.

9. A composition according to any preceding claim in which for the copolymer:
a ranges from 0.2 to 1 % by weight of the copolymer;
b ranges from 25 to 50 % by weight of the copolymer;
c ranges from 40 to 65 % by weight of the copolymer; and
d ranges from 2 to 12 % by weight of the copolymer.

10. A composition according to any preceding claim in which the level of copolymer is present at a level from 0.05 to 1 wt. % of the total composition.

11. A composition according to any preceding claim in which the particulate antidandruff agent comprises zinc pyrithione.

12. A composition according to any preceding claim in which the composition comprises at least 0.5 wt. % of the total composition of an anionic surfactant.

13. Use of a copolymer to deposit a particulate antidandruff agent to the scalp and to minimize deposition of the particulate antidandruff agent to the hair; the copolymer being the addition polymerization product of:
a) from 0.1 to 5 wt. % of a first unsaturated monomer **A** of a ethylenically unsaturated cis diacid of formula (I):
HOOC-CR₅=CR₆-COOH (I)
or an unsaturated cyclic anhydride precursor of such an ethylenically unsaturated diacid, the anhydride having formula (II) 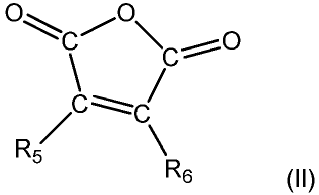 where R₅ and R₆ are individually selected from H, C₁-C₃ alkyl, phenyl, chlorine and bromine;
b) from 1 to 60 wt. % of a second ethylenically unsaturated monomer **B** selected from acrylic acid, methacrylic acid and combinations thereof,
c) from 30 to 75 wt. % of a (meth)acrylate monomer **C** selected from C₁ to C₈ alkyl esters of (meth)acrylic acid and C₁ to C₈ alkyl esters of methacrylic acid and combinations thereof
d) from 1 to 25 wt. % of an associative monomer **D** of the formula III: 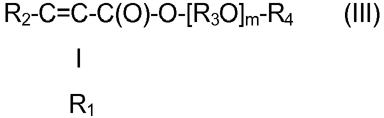
Where R₁ and R₂ are each independently selected from H, and 6₁₋₃ alkyl; R₃ is C₂-C₄ and mixtures thereof, preferably C₂;
m, the average number of alkoxy units R₃O, is from 6 to 40;
R₄ is alkyl or alkylaryl where the alkyl part is linear or branched; and the total number of carbons is from 6 to 40.

## Patentansprüche

1. Wässrige Haarpflegezusammensetzung, umfassend:
i) mindestens 0,01 Gew.-% der gesamten Zusammensetzung von einem teilchenförmigen Antischuppenmittel;
ii) mindestens 0,05 Gew.-% des Additionspolymerisationsprodukts von:
a) 0,1 bis 5 Gew.-% eines ersten ungesättigten Monomers A von einer ethylenisch ungesättigten Disäure der Formel (I):
HOOC-CR₅=CR₆-COOH (I)
oder einer ungesättigten cyclischen Anhydrid-Vorstufe einer solchen ethylenisch ungesättigten cis-Disäure, wobei das Anhydrid die Formel (II) aufweist 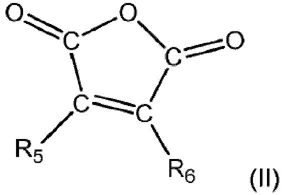 worin R₅ und R₆ individuell aus H, C₁-C₃-Alkyl, Phenyl, Chlor und Brom ausgewählt sind;
b) 1 bis 60 Gew.-% eines zweiten ethylenisch ungesättigten Monomers B ausgewählt aus Acrylsäure, Methacrylsäure und Kombinationen davon,
c) 30 bis 75 Gew.-% eines (Meth)acrylat-Monomers C ausgewählt aus C₁-bis C₈-Alkylestern von (Meth)acrylsäure und C₁- bis C₈-Alkylestern von Methacrylsäure und Kombinationen davon,
d) 1 bis 25 Gew.-% eines assoziativen Monomers D der Formel III: 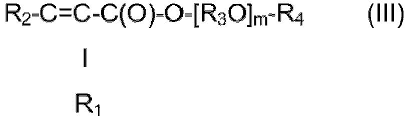
worin R₁ und R₂ jeweils unabhängig ausgewählt sind aus H und C₁₋₃-Alkyl; R₃ C₂-C₄ und Mischungen davon, bevorzugt C₂, ist;
m, die durchschnittliche Anzahl von Alkoxyeinheiten R₃O, 6 bis 40 ist;
R₄ Alkyl oder Alkylaryl ist, wobei der Alkylteil linear oder verzweigt ist; und die Gesamtzahl an Kohlenstoffen 6 bis 40 ist.

2. Zusammensetzung nach irgendeinem vorhergehenden Anspruch, in der das Monomer A ausgewählt ist aus der Gruppe bestehend aus Maleinsäureanhydrid, Maleinsäure und Kombinationen davon.

3. Zusammensetzung nach irgendeinem vorhergehenden Anspruch, in der das Monomer B Methacrylsäure ist.

4. Zusammensetzung nach irgendeinem vorhergehenden Anspruch, in der Monomer C des Copolymers ausgewählt ist aus der Gruppe bestehend aus Ethylacrylat, Methylacrylat, Ethylmethacrylat, Methylmethacrylat, Butylacrylat, Butylmethacrylat und Mischungen davon.

5. Zusammensetzung nach irgendeinem vorhergehenden Anspruch, in der Monomer C des Copolymers Ethylacrylat ist.

6. Zusammensetzung nach irgendeinem vorhergehenden Anspruch, in der Monomer D von der Formel iv ist 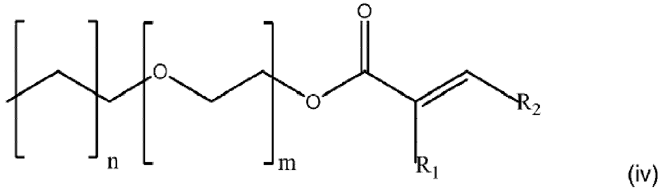 in der R₁ und R₂ jeweils unabhängig H, C₁- bis C₃-Alkyl sind; n im Bereich von 6 bis 40 liegt und m im Bereich von 6 bis 40 liegt.

7. Zusammensetzung nach irgendeinem vorhergehenden Anspruch, in der für Monomer D n im Bereich von 12 bis 22 liegt und m im Bereich von 20 bis 30 liegt.

8. Zusammensetzung nach irgendeinem vorhergehenden Anspruch, in der in Monomer D R₁ H oder Methyl ist und R₂ H ist.

9. Zusammensetzung nach irgendeinem vorhergehenden Anspruch, in der für das Copolymer:
a im Bereich von 0,2 bis 1 Gewichts-% des Copolymers liegt;
b im Bereich von 25 bis 50 Gewichts-% des Copolymers liegt;
c im Bereich von 40 bis 65 Gewichts-% des Copolymers liegt; und
d im Bereich von 2 bis 12 Gewichts-% des Copolymers liegt.

10. Zusammensetzung nach irgendeinem vorhergehenden Anspruch, in der der Gehalt des Copolymers in einer Menge von 0,05 bis 1 Gew.-% der gesamten Zusammensetzung vorhanden ist.

11. Zusammensetzung nach irgendeinem vorhergehenden Anspruch, in der das teilchenförmige Antischuppenmittel Zinkpyrithion umfasst.

12. Zusammensetzung nach irgendeinem vorhergehenden Anspruch, in der die Zusammensetzung mindestens 0,5 Gew.-% der Gesamtzusammensetzung von einem anionischen Tensid umfasst.

13. Verwendung eines Copolymers zur Abscheidung eines teilchenförmigen Antischuppenmittels auf der Kopfhaut und zur Minimierung der Abscheidung des teilchenförmigen Antischuppenmittels auf dem Haar; wobei das Copolymer das Additionspolymerisationsprodukt von:
a) 0,1 bis 5 Gew.-% eines ersten ungesättigten Monomers A von einer ethylenisch ungesättigten cis-Disäure der Formel (I):
HOOC-CR₅=CR₆-COOH (I)
oder einer ungesättigten cyclischen Anhydrid-Vorstufe einer solchen ethylenisch ungesättigten Disäure, wobei das Anhydrid die Formel (II) aufweist 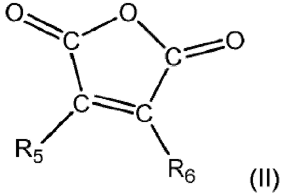 worin R₅ und R₆ individuell aus H, C₁-C₃-Alkyl, Phenyl, Chlor und Brom ausgewählt sind;
b) 1 bis 60 Gew.-% eines zweiten ethylenisch ungesättigten Monomers B ausgewählt aus Acrylsäure, Methacrylsäure und Kombinationen davon,
c) 30 bis 75 Gew.-% eines (Meth)acrylat-Monomers C ausgewählt aus C₁- bis C₈-Alkylestern von (Meth)acrylsäure und C₁- bis C₈-Alkylestern von Methacrylsäure und Kombinationen davon,
d) 1 bis 25 Gew.-% eines assoziativen Monomers D der Formel III: 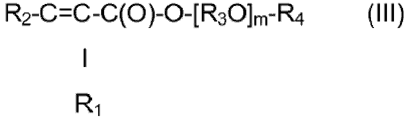
worin R₁ und R₂ jeweils unabhängig ausgewählt sind aus H und C₁₋₃-Alkyl; R₃ C₂-C₄ und Mischungen davon, bevorzugt C₂, ist;
m, die durchschnittliche Anzahl von Alkoxyeinheiten R₃O, 6 bis 40 ist;
R₄ Alkyl oder Alkylaryl ist, wobei der Alkylteil linear oder verzweigt ist; und die Gesamtzahl an Kohlenstoffen 6 bis 40 ist,
ist.

## Revendications

1. Composition aqueuse pour soins des cheveux comprenant :
i) au moins 0,01 % en poids de la composition totale d'un agent antipelliculaire particulaire ;
ii) au moins 0,05 % en poids du produit de la polymérisation par addition de :
a) de 0,1 à 5 % en poids d'un premier monomère **A** insaturé de type diacide à insaturation éthylénique de formule (I) :
HOOC-CR₅=CR₆-COOH (I)
ou d'un précurseur d'anhydride cyclique insaturé dudit cis-diacide à insaturation éthylénique, l'anhydride répondant à la formule (II) 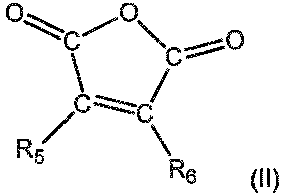 où R₅ et R₆ sont individuellement choisis parmi H, un alkyle C₁-C₃, un phényle, un atome de chlore et de brome ;
b) de 1 à 60 % en poids d'un second monomère **B** à insaturation éthylénique choisi parmi l'acide acrylique, l'acide méthacrylique et leurs combinaisons,
c) de 30 à 75 % en poids d'un monomère de (méth)acrylate C choisi parmi les esters d'alkyle C₁ à C₈ d'acide (méth)acrylique et les esters d'alkyle C₁ à C₈ d'acide méthacrylique et leurs combinaisons ;
d) de 1 à 25 % en poids d'un monomère associatif **D** de formule III : 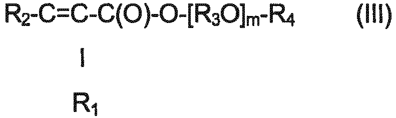
où R₁ et R₂ sont chacun indépendamment choisis parmi H et un alkyle C₁₋₃ ; R₃ est C₂-C₄ et leurs mélanges, de préférence C₂ ;
m, nombre moyen de motifs alcoxy R₃O, vaut de 6 à 40 ;
R₄ est un alkyle ou aralkyle dont la partie alkyle est linéaire ou ramifiée ; et
le nombre total de carbones est de 6 à 40.

2. Composition selon la revendication précédente dans laquelle le monomère A est choisi dans le groupe constitué par l'anhydride maléique, l'acide maléique et leurs combinaisons.

3. Composition selon l'une quelconque des revendications précédentes dans laquelle le monomère B est l'acide méthacrylique.

4. Composition selon l'une quelconque des revendications précédentes dans laquelle le monomère C du copolymère est choisi dans le groupe constitué par l'acrylate d'éthyle, l'acrylate de méthyle, le méthacrylate d'éthyle, le méthacrylate de méthyle, l'acrylate de butyle, le méthacrylate de butyle et leurs mélanges.

5. Composition selon l'une quelconque des revendications précédentes dans laquelle le monomère C du copolymère est l'acrylate d'éthyle.

6. Composition selon l'une quelconque des revendications précédentes dans laquelle le monomère D répond à la formule iv 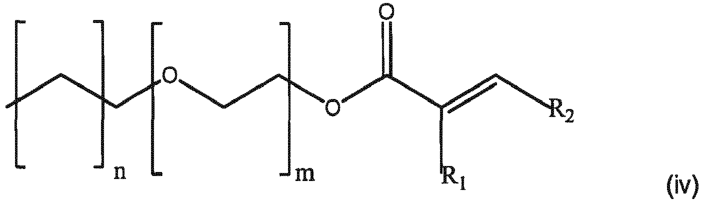 dans laquelle chaque R₁ et R₂ est indépendamment H, un alkyle C₁ à C₃ ; n vaut de 6 à 40 et m vaut de 6 à 40.

7. Composition selon l'une quelconque des revendications précédentes dans laquelle pour le monomère D, n vaut de 12 à 22 et m de 20 à 30.

8. Composition selon l'une quelconque des revendications précédentes dans laquelle dans le monomère D, R₁ est H ou un méthyle et R₂ est H.

9. Composition selon l'une quelconque des revendications précédentes dans laquelle pour le copolymère :
a représente de 0,2 à 1 % en poids du copolymère ;
b représente de 25 à 50 % en poids du copolymère ;
c représente de 40 à 65 % en poids du copolymère ; et
d représente de 2 à 12 % en poids du copolymère.

10. Composition selon l'une quelconque des revendications précédentes dans laquelle le copolymère est présent à un niveau de 0,05 à 1 % en poids de la composition totale.

11. Composition selon l'une quelconque des revendications précédentes dans laquelle l'agent antipelliculaire particulaire comprend de la pyrithione de zinc.

12. Composition selon l'une quelconque des revendications précédentes dans laquelle la composition comprend au moins 0,5 % en poids de la composition totale d'un tensioactif anionique.

13. Utilisation d'un copolymère pour déposer un agent antipelliculaire particulaire sur le cuir chevelu et réduire au minimum le dépôt de l'agent antipelliculaire particulaire sur les cheveux ; le copolymère étant le produit de la polymérisation par addition de :
a) de 0,1 à 5 % en poids d'un premier monomère **A** insaturé de type cis-diacide à insaturation éthylénique de formule (I) :
HOOC-CR₅=CR₆-COOH (I)
ou d'un précurseur d'anhydride cyclique insaturé dudit diacide à insaturation éthylénique, l'anhydride répondant à la formule (II) 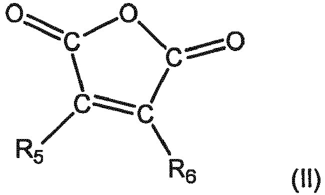 où R₅ et R₆ sont individuellement choisis parmi H, un alkyle C₁-C₃, un phényle, un atome de chlore et de brome ;
b) de 1 à 60 % en poids d'un second monomère **B** à insaturation éthylénique choisi parmi l'acide acrylique, l'acide méthacrylique et leurs combinaisons,
c) de 30 à 75 % en poids d'un monomère de (méth)acrylate C choisi parmi les esters d'alkyle C₁ à C₈ d'acide (méth)acrylique et les esters d'alkyle C₁ à C₈ d'acide méthacrylique et leurs combinaisons ;
d) de 1 à 25 % en poids d'un monomère associatif D de formule III : 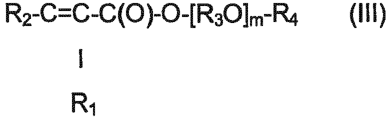
où R₁ et R₂ sont chacun indépendamment choisis parmi H et un alkyle C₁₋₃ ;
R₃ est C₂-C₄ et leurs mélanges, de préférence C₂ ;
m, nombre moyen de motifs alcoxy R₃O, vaut de 6 à 40 ;
R₄ est un alkyle ou aralkyle dont la partie alkyle est linéaire ou ramifiée ; et le nombre total de carbones est de 6 à 40.
